# EUROPEAN PATENT APPLICATION

(11) **EP 2 671 450 A1**
(43) Date of publication of application: **11.12.2013**
(21) Application number: 12170826.7
(22) Date of filing: 05.06.2012
(51) Int. Cl.: A01N 59/16, A61K 33/38, B22F 1/00, B22F 9/24, B82Y 5/00

(54) **Method for preparing nanoparticles, nanoparticles obtained thereby and use thereof**

(71) Applicant: King Saud University, 11421 Riyadh (SA)
(72) Inventor: Al-Enazi, Nouf Mohammed, Riyadh 11333 (SA); Khlifa, Neveen Abdel-Raouf Mohammed, Riyadh 11333 (SA); Al-Othman, Monerah Rashed, Riyadh 11333 (SA); Hindi, Awatif A., Riyadh 11333 (SA)
(74) Representative: Goddar, Heinz J.

(57) **Abstract**

The present invention relates to a method for preparing nanoparticles by using an algal extract, nanoparticles prepared thereby and their use as antibacterial agents.

## Description

The present invention relates to a method for preparing nanoparticles, nanoparticles obtained thereby and their use as antibacterial agent.

Nanotechnology, in particular the synthesis and use of nanoparticles, is introduced in many fields and features a plurality of possible applications such as chemical or biological detectors, drug delivery systems, new generation of laser, reinforced materials, thermal barriers and inkjet systems as well as textiles.

Most of the known synthetic methods for the production of nanoparticles relay on the reaction of toxic reducing agents, such as hydrazine, DMF, sodium borohydride, etc. and suitable nanoparticle precursors in the presence of separate stabilizing agents. Most of the generally used reducing agents are toxic and feature both potential environment and biological risks. Besides this, said approaches cause high costs and are therefore not favorable for an industrial application.

Moreover, in the recent years several substantiated hints indicate that nanoparticles can compromise the health of living organism by penetrating the cells of plants as well as of animals. Accordingly, several so far unknown risks, in particular when using nanoparticles comprising toxic materials, have to be assumed.

Accordingly, there is an urgent need of new strategies for synthesizing nanoparticles by using non-toxic, natural sources.

Armendarez et al., Journal of Nanoparticle Research, 2004, 6, 377 - 382 discloses a size controlled gold nanoparticle formation by a biomass and the use of plants in nanobiotechnology. Among several different approaches, the formation of gold nanoparticles by using dead and live tissues of alfalfa, hops, fungus and algae is described.

It is an object of the present invention to provide a synthesis of nanoparticles from easy available, non-toxic, natural precursors. Moreover, it is an object to provide a less energy consuming, cheap synthesis, allowing easy control of the nanoparticle properties, like shape, size distribution etc.

These objects are achieved by a method for preparing nanoparticles, comprising the steps:
a) providing an extract from at least one alga;
b) adding at least one nanoparticle precursor to the extract;
c) mixing the nanoparticle precursor and the extract to obtain nanoparticles stabilized by at least parts of the constituents of the extract.

In a preferred embodiment the extract is an organic extract, preferably is a chlorinated hydrocarbonic and/or alcoholic extract, more preferably a chloroformic and/or ethanolic extract.

Preferably, the alga is a marine macroalga, preferably is selected from the group consisting of *Padina pavonia, Laurencia sp.* and/or *Scinaia pseudocrispa.*

In a further preferred embodiment, the nanoparticle precursor is an inorganic metal salt and/or an inorganic acid containing a metal.

More preferably, the nanoparticles are metal nanoparticles.

In one embodiment, the nanoparticles are silver and/or gold nanoparticles.

In a most preferred embodiment, the nanoparticle precursor is chloroauric acid and/or silver nitrate.

The object is further achieved by nanoparticles prepared by the inventive method.

It is preferred, that the nanoparticles have a spherical, triangular, hexagonal or square shape.

The nanoparticles have preferably a size of 5 - 1.000 nm, preferably 10 - 500 nm.

The object is finally achieved by gold and/or silver nanoparticles prepared by the inventive method for use as antibacterial agent.

Surprisingly, it was found that the inventive method provides the possibility to synthesize nanoparticles in an easy, cost efficient way from non-toxic, natural materials, in particular by using algal extracts as reduction and stabilizing agent. Moreover, it was surprisingly found that the inventive method allows faster nanoparticle growth, the possibility to achieve a variety of particle shapes and a better control of the particle size distribution, compared to the prior art.

Those skilled in the art will be aware, that the inventive method will preferably further comprise a claiming operation step to remove free biomass residue and/or compounds not bound to the surface of the nanoparticles. This removal can, for example, be conducted by centrifugal separation of the prepared nanoparticles from the supernatant.

"Organic extract" in terms of the present invention means an extract obtained by the use of an organic solvent. The extracts can be obtained by using standard extraction techniques, like using a separatory funnel, a soxhlet apparatus etc. One or more different organic solvents together can be used for this purpose. Furthermore, the extraction can comprise one or more different extraction steps in which the same or different organic solvents and extraction technique are used.

As a matter of course, it will be understood by those skilled in the art that in case that an inorganic salt is used as nanoparticle precursor, this salt will usually comprise a metal ion from which the nanoparticles can be achieved.

The use of silver nanoparticles prepared by the inventive method as antibacterial agent comprises the use against gram-positive as well as against gram-negative bacteria. The nanoparticles can be used as antibacterial agents in a variety of applications, such as antibacterial coatings on household items, in plasters or bandages, on food packages, in cosmetics or in pharmaceutical compositions.

The macroalgal species to be extracted, can be collected in large amounts from nature, for example from the beaches of Saudi Arabia.

Further advantages and features of the inventive method, the nanoparticles prepared thereby and the use thereof can be taken from the following examples with reference to the accompanying figures wherein
Fig. 1 shows a Transmission Electron Microscope (TEM) image of a triangular shaped gold nanoparticle formed by using an ethanolic extract of alga *Padina pavonia;*
Fig. 2 shows hexagonal shaped gold nanoparticles formed by using an ethanolic extract of alga *Padina pavonia;*
Fig. 3 shows spherical silver nanoparticles formed by using an ethanolic extract of alga *Padina pavonia;*
Fig. 4 shows the inhibition zone around disks saturated by gold and silver nanoparticles prepared from a powder (P.P.), as a reference, and an ethanolic extract (P.Ex.) of *Padina pavonia* against *Staphylococcus aureus;*
Fig. 5 shows the inhibition zone around disks saturated by gold and silver nanoparticles produced by a powder (P.P.), as a reference, and an ethanolic extract (P. Ex.) of *Padina pavonia* against *Klebsielle pneumoniae;*

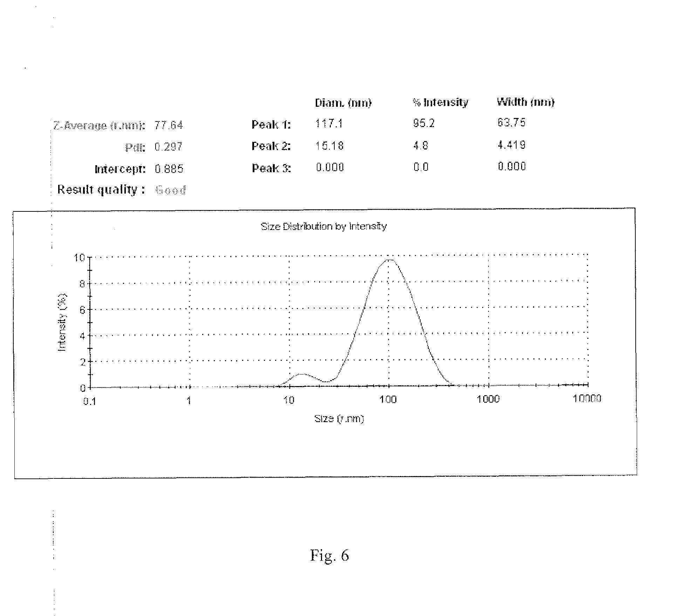

Fig. 6 shows the size distribution of gold nanoparticles obtained by using an algal extract, wherein the particle sizes were determined by measuring zeta potential;

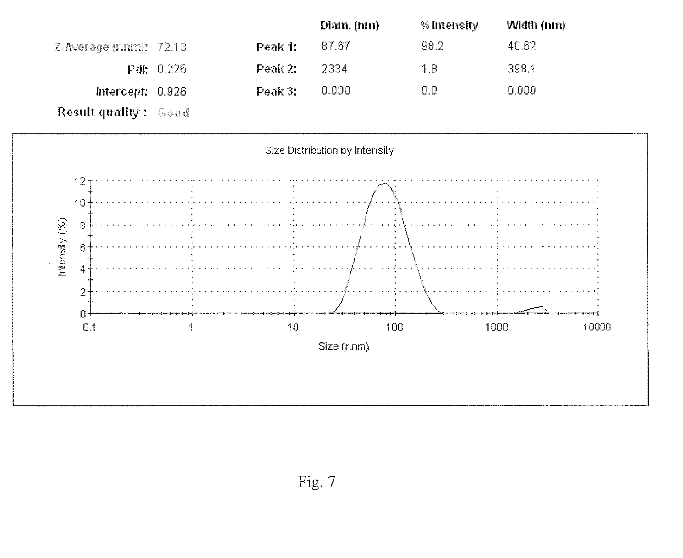

Fig. 7 shows the size distribution of silver nanoparticles obtained by using an algal extract obtained from *Padina pavonia,* wherein the particle sizes were determined by measuring zeta potential;

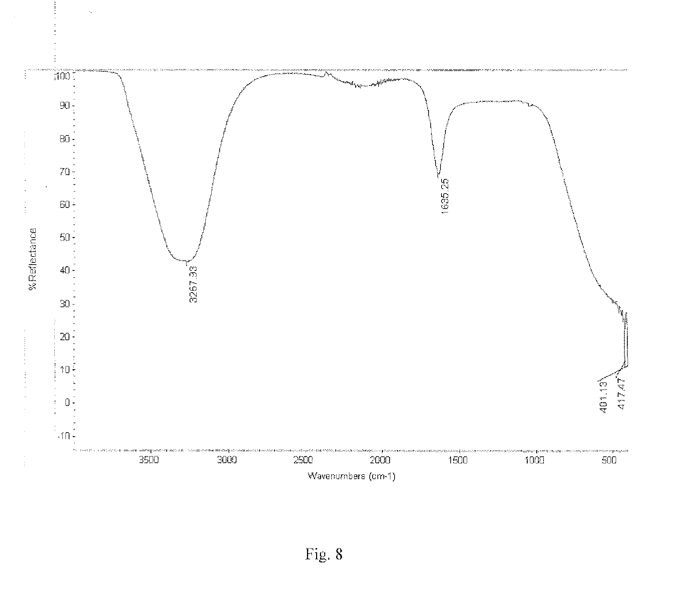

Fig. 8 shows an FTIR spectrum of gold nanoparticles synthesized from an ethanolic extract of *Padina pavonia;*

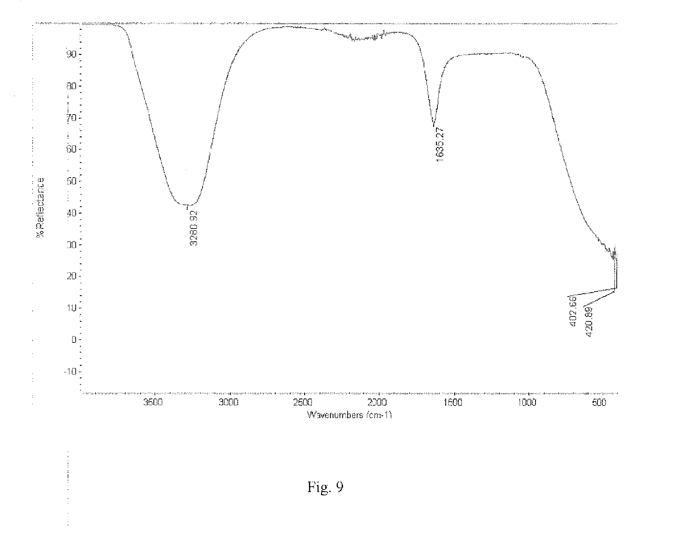

Fig. 9 shows an FTIR spectrum of silver nanoparticles synthesized from an ethanolic extract of *Padina pavonia.*

### Example 1

Algal material of *Padina pavonia, Laurencia sp. or Scinaia Pseudocrispa* was dried for 2 days at 70°C. The dried material was ground to achieve an algal powder. Afterwards, the algal powder was extracted using ethanol or chloroform in a soxhlet extractor for 20 hours. The extraction step was repeated three times. After combining of all extracts, the solvent was evaporated under vacuum at 50°C.

1 ml of an ethanolic or chloroformic algal extract was added to 100 ml of an aqueous solution of HAuCl₄ or AgNO₃ and stirred for about 5 minutes. A color change, from yellow transparent to red purple in case of gold and from transparent to brown in case of silver, indicted the formation of the respective nanoparticles. To remove free biomass residues or compounds were not attached to the nanoparticles, the residual solution of 100 ml after reaction was centrifuged at 5.000 rpm for 10 min.

The formed nanoparticles were analyzed by Transmission Electron Microscopy (TEM) (JEM-1011, JEOL, Japan), UV-Vis spectroscopy (Lambda 25, PerkinElmer, United Kingdom), FTIR spectroscopy (NICOLET 6700, Thermo, USA) and measurement of the Zeta Potentials using a Zetasizer (ZEN 3600, MALVERN, United Kingdom).

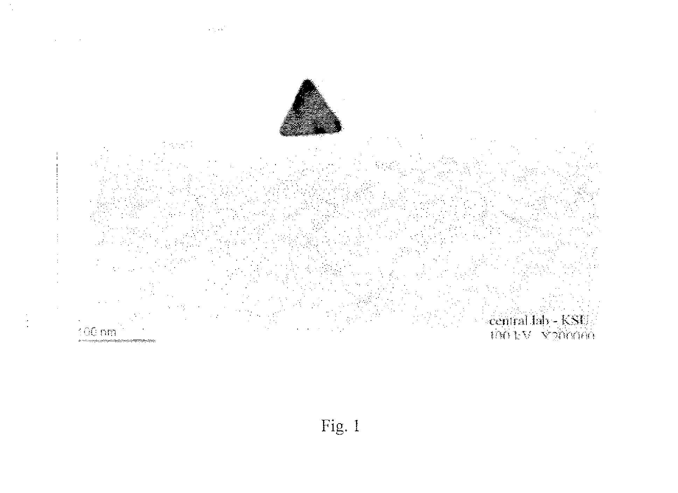

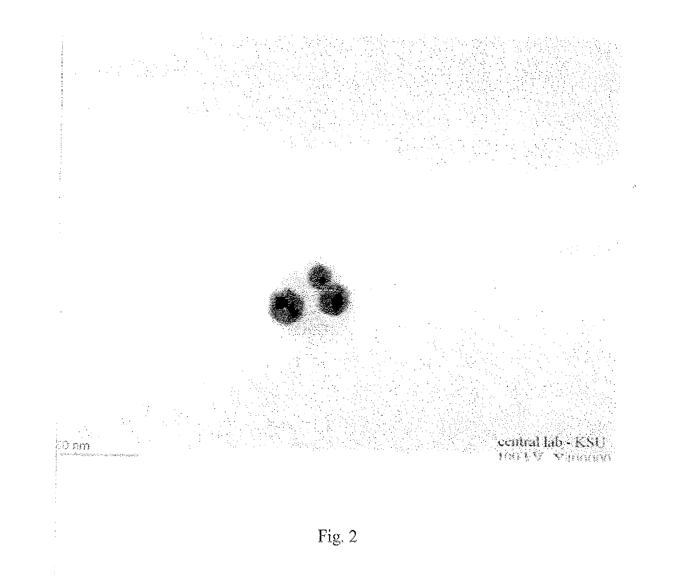

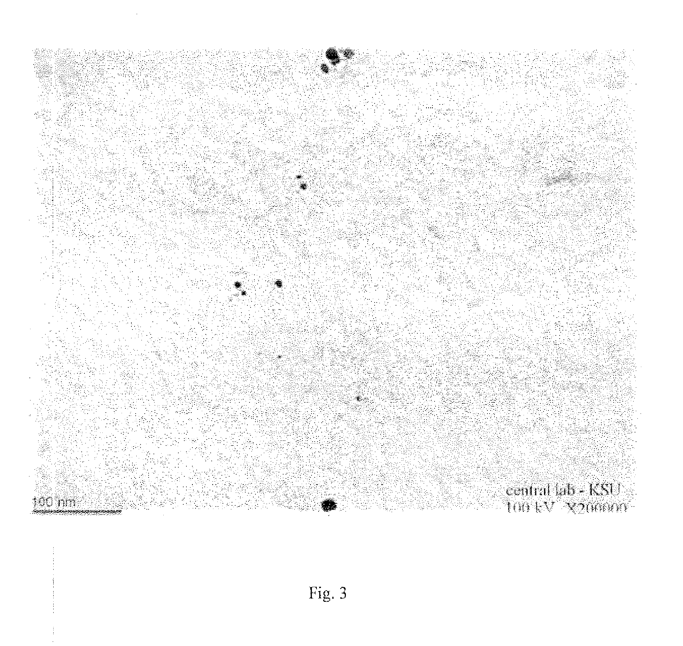

As shown in Fig. 1 to 3, nanoparticles of different shapes (spherical, triangular, hexagonal and square) were prepared this way.

The shape of the prepared nanoparticles depends on the algae species used in the preparation process, in particular on the functional groups of the compounds contained in the algal extracts, on the retention reaction time and on the concentration and type of the bioactive compounds of the algal extract.

The average size of the produced gold nanoparticles was 77 nm. Silver nanoparticles prepared this way featured an average size of 72 nm.

### Example 2

Antibacterial screening of the obtained gold and silver nanoparticles:

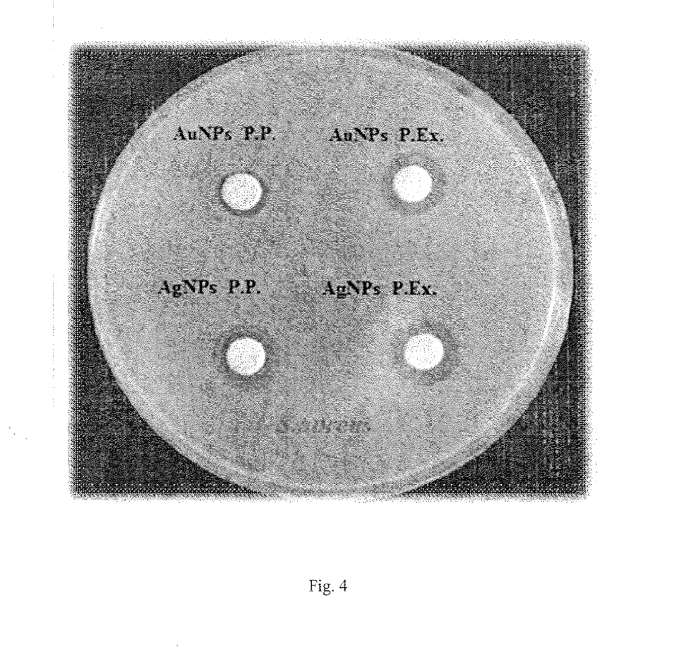

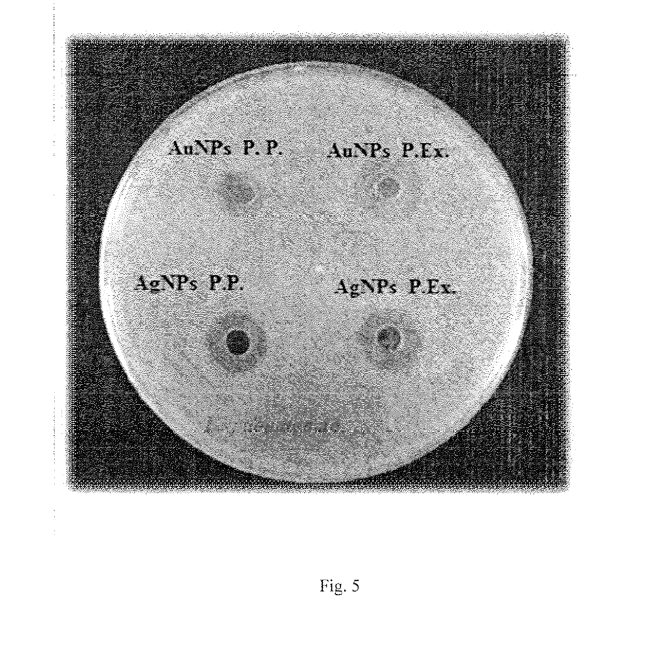

The antibacterial test of both gold and silver algal nanoparticles prepared according to example 1, show a significant inhibition against both gram-positive and gram-negative bacteria, as shown in Fig. 4 and Fig. 5. As a reference, nanoparticles were also prepared by using an alga powder as described in the prior art, instead of an extract. The superiority of the inventive method can be taken from comparison of the different antibacterial effects shown in Fig. 4 and Fig. 5.

The features disclosed in the foregoing description and in the claims and the drawings may, both separately and in any combination thereof, be material for realizing the invention in diverse forms thereof.

## Claims

1. Method for preparing nanoparticles, comprising the steps:
d) providing an extract from at least one alga;
e) adding at least one nanoparticle precursor to the extract;
f) mixing the nanoparticle precursor and the extract to obtain nanoparticles stabilized by at least parts of the constituents of the extract.

2. Method according to claim 1, wherein the extract is an organic extract, preferably is a chlorinated hydrocarbonic and/or alcoholic extract, more preferably a chloroformic and/or ethanolic extract.

3. Method according to claim 1 or 2, wherein the alga is a marine macroalga, preferably is selected from the group consisting of *Padina pavonia, Laurencia sp.* and/or *Scinaia pseudocrispa.*

4. Method according to any of the preceding claims, wherein the nanoparticle precursor is an inorganic metal salt and/or an inorganic acid containing a metal.

5. Method according to any of the preceding claims, wherein the nanoparticles are metal nanoparticles.

6. Method according to any of the preceding claims, wherein the nanoparticles are silver and/or gold nanoparticles.

7. Method according to any of the preceding claims, wherein the nanoparticle precursor is chloroauric acid and/or silver nitrate.

8. Nanoparticles prepared by a method according to any of the preceding claims.

9. Nanoparticles according to claim 8, wherein the nanoparticles have a spherical, triangular, hexagonal or square shape.

10. Nanoparticles prepared by a method according to claim 6 for use as antibacterial agent.

## Amended claims

### Amended claims in accordance with Rule 137(2) EPC.

**1.** Method for preparing nanoparticles, comprising the steps:
a) providing an extract from at least one alga;
b) adding at least one nanoparticle precursor to the extract;
c) mixing the nanoparticle precursor and the extract to obtain nanoparticles stabilized by at least parts of the constituents of the extract, wherein the alga is se-elected from the group consisting of *Padina pavonia, Laurencia sp.* and/or *Sci-naia pseudocrispa.*

**2.** Method according to claim 1, wherein the extract is an organic extract, preferably is a chlorinated hydrocarbonic and/or alcoholic extract, more preferably a chloroformic and/or ethanolic extract.

**3.** Method according to claim 1 or 2, wherein the nanoparticle precursor is an inorganic metal salt and/or an inorganic acid containing a metal.

**4.** Method according to any of the preceding claims, wherein the nanoparticles are metal nanoparticles.

**5.** Method according to any of the preceding claims, wherein the nanoparticles are silver and/or gold nanoparticles.

**6.** Method according to any of the preceding claims, wherein the nanoparticle precursor is chloroauric acid and/or silver nitrate.

**7.** Nanoparticles prepared by a method according to any of the preceding claims.

**8.** Nanoparticles according to claim 7, wherein the nanoparticles have a spherical, triangular, hexagonal or square shape.

**9.** Nanoparticles prepared by a method according to claim 5 for use as antibacterial agent.
